Europäisches Patentamt

European Patent Office

Office européen des brevets

(11) Publication number: **0 096 477**
**B1**

# EUROPEAN PATENT SPECIFICATION

(45) Date of publication of patent specification: **10.12.86**

(51) Int. Cl.⁴: **A 23 C 21/02, C 12 P 7/52**

(21) Application number: **83302717.0**

(22) Date of filing: **13.05.83**

(54) Whey fermentation process.

(30) Priority: **07.06.82 US 385627**

(43) Date of publication of application:
**21.12.83 Bulletin 83/51**

(45) Publication of the grant of the patent:
**10.12.86 Bulletin 86/50**

(84) Designated Contracting States:
**AT BE CH DE FR GB LI NL SE**

(56) References cited:
**US-A-1 470 885**
**US-A-2 555 514**
**US-A-3 466 176**

**JOURNAL OF DAIRY SCIENCE, vol. 61, no. 6,
June 1978, pages 710-713; T. MELO et al.:
"Effect of ultra-high-temperature steam
injection on model systems of alpha-
lactalbumin and beta-lactoglubulin"**

(73) Proprietor: **STAUFFER CHEMICAL COMPANY
Westport Connecticut 06880 (US)**

(72) Inventor: **Pyne, Charles Herbert
264 Half Mile Road
Southport Connecticut, 06490 (US)**
Inventor: **Schwartz, Robert David
3042 Santa Maria Court
Concord California (US)**

(74) Representative: **Smith, Sydney et al
Elkington and Fife High Holborn House 52/54
High Holborn
London WC1V 6SH (GB)**

(56) References cited:

**JOURNAL OF DAIRY SCIENCE, vol. 64, no. 4,
April 1981, pages 581-587; W.O. McCARTY et
al.: "Effects of ultrahigh temperature steam
injection processing on composition of
carbonyls in milk fat"**

Courier Press, Leamington Spa, England.

**Description**

This invention relates to an improved fermentation process, particularly for the fermentation of whey.

Whey is a naturally occurring substance which is the serum or watery part of milk which is separated from the curds in the process of making cheese. Dried whey comprises approximately 13% protein and 73% lactose, and the balance inorganic salts. One of the uses of whey in commerce involves fermenting it and incorporating it into bakery products, where it acts as a mycostatic agent, thus enabling the products to have substantially longer shelf lives than would otherwise be possible. The active mycostatic agent in whey appears to be propionic acid which is produced as a consequence of the fermentation process.

Suggested bacteria for fermentation, as disclosed in U.S. Patents, 1,910,130 and 1,898,320, include *Bacterium acidi-propionici*. In producing fermented whey, it is desirable to ferment it with a known culture in order to keep out pathogens, and control the product quality and specifications. One of the steps in the process of fermentation is the sterilization of the whey substrate, which must be done without adversely effecting the subsequent fermentation. Sterilization is necessary in order to kill any residual organisms in the whey prior to introducing the organism for fermentation.

The bacterium organism of choice for the fermentation is a strain of *Propionibacterium shermanii*. In the normal prior art process, the whey is sterilized by autoclaving at 120°C, at 15 psi, (1.03 bar) for 15 minutes. The concentration of the whey component being sterilized is approximately 2% by weight. It has been found that when whey having a solids concentration of more than approximately 2% is sterilized by autoclave, successful subsequent fermentation with the *Propionibacterium shermanii* is somehow prevented. It is desirable to raise the solids concentration of the whey being sterilized so as to provide a high solids broth that increases the concentration of propionic acid produced in the fermentation process, and also to produce a concentration that is easier to process downstream, as by air drying.

The process of this invention is therefore concerned with an improved process for sterilizing whey which allows sterilization of whey having a solids concentration of substantially higher degree than has previously been possible, yet still allows the sterilized whey to be fermented by the standard bacterium. Because the higher solids concentration is permitted, a greater quantity of propionic acid is produced and a more effective mycostat is obtained.

We have now discovered an improved process for fermenting whey producing high concentrations of propionic acid in a sterile medium. The process comprises the steps of subjecting a quantity of whey solution having a solids content of from 7 to 12% (weight/volume) to a pasteurization treatment consisting of heating said whey solution to a temperature of 90°C for 60 seconds, and thereafter subjecting said whey solution to a direct steam injection while being passed through a transport mechanism wherein the steam has a temperature high enough to produce a medium temperature for the solution of at least 140°C, for a period of time within the range of 4 to 12 seconds, whereby a sterile whey solution of high solids content is produced which can be subsequently fermented by *Propionibacterium shermanii* to produce high concentrations of propionic acid.

The purpose of the pasteurization step is to partially denature the proteins in the whey solution, because it has been found that unless these proteins are at least partially denatured, when the whey solution is subjected to the steam treatment the proteins in it will coagulate and stick to the transport mechanism, thereby fouling the mechanism. With the partial denaturization of the proteins with the pasteurization step, it has been found that the whey material can be subsequently sterilized by the steam injection as hereinafter described without substantial complications caused by the protein material sticking to the transport mechanism.

When the transport mechanism is fouled by protein, it causes frequent shutdowns and drives up labour costs unnecessarily.

While the whey solution can be subjected to the direct steam injection by any suitable means, it has been found convenient to conduct said process step in an Alpha-Laval sterilizer. This sterilizer is commercially available and is manufactured by the Alpha-Laval Company, Lund, Sweden. The preferred model is capable of processing about 3000 gallons (11.356 m³) per hour of solution.

The following Examples illustrate the invention.

Example 1

A quantity of a solution of whey, approximately 200 litres, was obtained (the solution was a 7.5% solids solution of whey obtained from a local dairy) and placed in a storage container. Thereafter the whey solution was adjusted to a pH of 7 with NaOH, and then was pumped through a Contherm scraped surface heat exchanger maintained at 90°C, and the duration of the transport was 60 seconds. Thereafter the whey solution was then pumped into another storage container and cooled to 20°C. From the storage container, the whey solution was pumped through an Alpha-Laval steam injector at the rate of 115 l per hour. While passing through a hold tube in the Alpha-Laval sterilizer, the whey solution was subjected to a direct steam injection for a period of approximately 4 seconds. Thereafter, it was pumped through a venturi nozzle for cooling and subsequently stored in an aseptic container.

The whey solution was later inoculated with a bacterium and fermented in a conventional manner.

The advantage of the process of the prevent invention over the process as practised previously, is demonstrated in Table I below, wherein various solutions containing differing quantities of whey solids

2

were sterilized by an autoclave method (120°C, 15 psi, [1.03 bar], 15 minutes) and by a direct steam method (Alpha-Laval sterilizer), and the solids later fermented with *Propionibacterium shermanii* and the quantity of propionic acid that was produced was measured. The results obtained for the autoclave method of sterilization are for comparative purposes.

TABLE I

| Whey solids in medium, % | Type of sterilization | HPr* Produced in P. shermanii fermentation, % |
|---|---|---|
| 7 | Autoclaved | <0.1 |
|  | DSI[1] | 1.3 |
| 12 | Autoclaved | <0.1 |
|  | DSI | 2.0 |

[1] DSI, direct steam injection
* Propionic acid

The entire fermentation process, which exemplifies the process of the invention, is conducted as follows.

A predetermined quantity of a whey-based medium (7% whey solids, 0.5% yeast extract) is obtained and partially denatured by heating to 90°C for 60 seconds, then subjected to a direct steam injection in an Alpha-Laval sterilizer for a period of from 4 to 10 seconds. Thereafter, the whey medium is inoculated with a culture of *P. shermanii* and incubated for 66 hours at 35°C. The pH of the fermentation broth is controlled at 7.0 by periodic addition of 50% sodium hydroxide.

After incubation, a quantity of the whey medium is obtained and analyzed by gas chromatography for the presence of propionic acid or in salt form, sodium propionate.

In the process of the invention it is essential, in order for a high solids content whey medium to be produced which can be sterilized and yet during fermentation produce the desired propionic acid, that the direct steam injection be at a temperature high enough to produce a medium temperature of at least 140°C, and for a period of time within the range of 4 to 12 seconds, and preferably 4 seconds.

As hereinbefore indicated, a suitable vehicle to accomplish the direct steam injection is the Alpha-Laval sterilizer, and this is suitable to process any amount of whey. If very large quantities are to be sterilized at one time, then obviously a larger sterilizer will be needed. The required scale-up would be obvious to those skilled in the art.

It has been found that if the whey medium is subjected to the sterilization procedure for less than 4 seconds, then inadequate sterilization is achieved, while if it is subjected to the direct steam injection for more than 12 seconds, viability of fermentation to produce propionic acid is reduced substantially.

It will be appreciated by those skilled in the art that the duration of sterilization treatment will depend to some extent upon the amount of substrate being treated, but the critical requirement is that the temperature of the direct steam injection raise the temperature of the medium at least to 140°C in order for the sterilization to be achieved. While the steam treatment can extend up to 20 seconds, there is a substantial risk that the ability of the bacteria to produce propionic acid will be eliminated.

Example 2

A quantity of dried whey, Teklac (20 grams), manufactured by the Foremost Company, is admixed with 10 g of yeast extract (KAT), manufactured by DiFco Company (Detroit, Michigan) per 1 litre of deionized water. The solution is adjusted to a pH of 7.0 with 50% sodium hydroxide and the solution was heated to 90°C for 60 seconds, then processed through the Alpha-Laval sterilizer. Inside the sterilizer, the solution was subjected to direct steam injection for approximately 4 seconds to sterilize it, and then removed. Thereafter, it was inoculated with a culture of *P. shermanii*. The fermentation broth containing the whey was then incubated for 42 hours at 30°C.

A sample of the medium was removed, and subjected to gas chromatography analysis for the production of propionic acid. Gas chromatographic techniques showed that the sterilized whey medium did in fact produce propionic acid in substantial quantities.

Fermentation with *Propionibacterium shermanii* of a whey broth comprising unhydrolyzed whey (acid or sweet), and yeast extract results in propionic acid formation and functionalization of the whey so that the whey product can be utilized as a food ingredient. This anaerobic fermentation can be carried out preferably in a pH range of 6 to 8, most preferably with the pH maintained in the range of from 6.5 to 7.5. The fermentation can be carried out at a temperature of from 25 to 35°C, preferably from 28 to 33°C. Typical composition of Teklac (sweet dairy whey) is as follows:

3

# 0 096 477

Chemical and physical specifications

Ingredient Listing: Whey

Typical Proximate Analysis

| | |
|---|---|
| Protein (N×6.38%) | 12.7 |
| Fat % | 1.1 (1.25% Maximum) |
| Moisture % | 4.5 (5.0% Maximum) |
| Ash % | 8.0 |
| Lactose % | 71.3 |
| Calories, Cal/100 g | 350.0 |

Typical Vitamin & Mineral Analysis

| | |
|---|---|
| Vitamin A I.U/100 g | Nil |
| Vitamin C mg/100 g | Nil |
| Thiamin mg/100 g | 0.40 |
| Riboflavin mg/100 g | 1.76 |
| Niacin mg/100 g | 1.00 |
| Calcium % | 0.71 |
| Iron % | Nil |
| Vitamin $B_{12}$ ug/100 g | 2.12 |
| Phosphorus % | 0.69 |
| Pantothenic Acid mg/100 g | 4.09 |

Microbiological Standards

| | |
|---|---|
| Standard Plate Count | 10,000/g (Maximum) |
| Coliforms | 9/g (Maximum) |
| E. coli | Negative |
| Salmonella | Negative |

The nutritional values listed above are within 80% of the value declared in compliance with Federal Nutritional Regulations 21 CFR S1.17(4) (ii).

| | Typical range | Limit |
|---|---|---|
| Solubility Index | 0.1—0.5 ml | 1.25 ml Max. |
| Acidity | 0.10—0.14% | 0.16 Max. |
| Alkalinity of Ash | 175—200 ml | 225 ml Max. |
| Scorched Particles | 7.5 mg | 15.0 mg Max. |
| Particle size (Through 40 Mesh −0.42 mm) | 99—100% | 98% Min. |

Concentration of whey can range preferably from 7.5% to 12%, and the additional yeast extract in the fermentation broth can range from 0.1 to 1%, preferably from 0.1% to 0.5%. Adequate propionic acid concentrations are usually reached within hours. All of the above weight percents are in weight per volume.

4

**0 096 477**

The propionic acid is actually produced by the fermentation of the lactose constituent in whey. Lactose, as has been previously indicated, is present in dried whey, in an amount of approximately 73%.

As previously stated, the fermented whey is used as a mycostatic agent and incorporated into breads, pastries, and other bakery products.

## Claims

1. A method for producing high concentrations of propionic acid from fermented whey comprising:
1) providing a quantity of a solution of whey having a solids content of from 7 to 12% (weight volume);
2) partially denaturing the protein content of said whey by subjecting said quantity to a temperature of 90°C for 60 seconds;
3) subjecting said solution to a direct steam injection while on a transport mechanism for a period of time ranging from 4 to 12 seconds so that the steam has a temperature high enough to produce a medium temperature of at least 140°C;
4) inoculating said whey with a propionic acid-producing bacterium viz. *Propionibacterium shermanii;*
5) incubating said solution of whey and said bacterium for a period of time and at a temperature sufficient to allow said medium to ferment to its maximum degree; and
6) thereafter drying said whey.

2. A method as claimed in claim 1 characterized in that the whey medium is a solution which contains 0.1 to 1% (weight/volume) of yeast as a fermenting agent.

## Patentansprüche

1. Verfahren zur Herstellung von Propionsäure in hohen Konzentrationen aus fermentierter Molke, dadurch gekennzeichnet, daß man

1) eine Menge einer Lösung an Molke mit einem Feststoffgehalt von 7 bis 12% (Gewicht/Volumen) verwendet,
2) den Proteingehalt dieser Molke teilweise denaturiert, indem man diese Menge einer Temperatur von 90°C während 60 Sekunden aussetzt,
3) die Lösung der direkten Dampfinjektion, während sie sich auf einem Transportmechanismus befindet, während einer Zeit im Bereich von 4 bis 12 Sekunden unterwirft, wobei der Dampf eine Temperatur besitzt, die hoch genug ist, eine mittlere Temperatur von mindestens 140°C zu ergeben,
4) die Molke mit einem Propionsäure erzeugenden Bakterium viz. *Propionibacterium shermanii* inokuliert,
5) die Lösung der Molke und des Bakteriums während einer Zeit und bei einer Temperatur inkubiert, die ausreichen, daß das Medium bis zum Maximalgrad fermentiert wird und
6) anschließend die Molke trocknet.

2. Verfahren nach Anspruch 1, dadurch gekennzeichnet, daß das Molkemedium eine Lösung ist, welche 0.1 bis 1% (Gewicht/Volumen) Hefe als Fermentationsmittel enthält.

## Revendications

1. Procédé d'obtention de concentrations élevées en acide propionique à partir de petit lait fermenté consistant:

(1) à réaliser une solution de petit lait dont la teneur en matières sèches soit comprise entre 7 et 12% (poids/volume);
(2) à dénaturer partiellement les protéines contenues dans ce petit lait en les soumettant à une température de 90°C pendant 60 secondes;
(3) à soumettre cette solution à une injection directe de vapeur dans un mécanisme de transport pendant une période de temps comprise entre 4 et 12 secondes, de telle sorte que la vapeur ait une température suffisamment élevée pour conférer une température d'au moins 140°C;
(4) à inoculer ce petit lait par une bactérie de formation d'acide propionique, par exemple Propionibacterium shermanii;
(5) à faire incuber cette solution de petit lait et cette bactérie pendant une période de temps et une température suffisantes pour permettre à ce milieu de fermenter à son degré maximum; et
(6) ensuite à sécher ce petit lait.

2. Procédé selon la revendication 1, caractérisé en ce que le milieu à base de petit lait est une solution qui contient de 0,1 à 1% (poids/volume) de levure comme agent de fermentation.